# DEMANDE DE BREVET EUROPEEN

(11) **EP 2 018 891 A1**
(43) Date de publication de la demande: **28.01.2009**
(21) Numéro de dépôt: 08160595.8
(22) Date de dépôt: 17.07.2008
(51) Int. Cl.: A61Q 19/00, A61K 8/99, A61P 17/00, A61P 17/08, A61K 35/08, A61K 35/74

(54) **Utilisation d'extraits bactériens cultivés sur eau thermale comme agent anti-rougeur**

(30) Priorité: 17.07.2007 FR 0756532
(71) Demandeur: L'Oréal, 75008 Paris (FR)
(72) Inventeur: Gueniche, Audrey, 92500 Rueil Malmaison (FR); Breton, Lionel, 78000 Versailles (FR)
(74) Mandataire: Allab, Myriam

(57) **Abrégé**

La présente invention se rapporte à l'utilisation d'extraits bactériens cultivés sur eau thermale comme agent réduisant les rougeurs de la peau telles que celles liées aux stress de l'environnement, ou encore les rougeurs associées à un désordre cutané par exemples celles survenant au cours de la rosacée.

Préférentiellement l'invention est un extrait issu de la bactérie *Vitreoscilla filiformis*, en particulier, la souche ATCC 15551, cultivées sur un milieu enrichi en eau de la Roche Posay.

## Description

La présente invention se rapporte à l'utilisation d'extraits bactériens cultivés sur eau thermale comme agent réduisant les rougeurs de la peau telles que celles liées aux stress de l'environnement, ou encore les rougeurs associées à un désordre cutané par exemples celles survenant au cours de la rosacée.
Préférentiellement, l'extrait bactérien est obtenu à partir de la bactérie *Vitreoscilla filiformis*, en particulier, la souche ATCC 15551, cultivées sur un milieu enrichi en eau de la Roche Posay.

La rosacée est une pathologie cutanée se manifestant en 4 sous-types : erythro-teliengectasique, papullo-pustuleuse, hypertrophique et oculaire. Dans les formes vasculaires, les capillaires sont dilatés, la continuité de l'endothélium étant parfois interrompu. Cela entraîne un oedème superficiel associé à une inflammation perivasculaire et périfolliculaire d'intensité moyenne. Au total, les éléments morphologiques suggèrent que le *primum movens* de la maladie est une altération vasculaire suivie d'une augmentation de la colonisation par la mite *Demodex*. Les estimations françaises sont que la prévalence de cette affection dans la population est de 3% dont 2/3 de femmes.

Certains neuropeptides comme la substance P ou le VIP contribuent au phénomène d'inflammation locale cause de rougeurs de la peau et chez les sujets atteints de rosacée.
Ainsi il demeure utile de disposer d'un produit qui agit sur les phénomènes cutanés liés à la libération de substance P, permettant ainsi de limiter l'action amplificatrice de la substance P sur le « process » inflammatoire et de réduire les érythèmes de la peau notamment observées chez les personnes atteintes de rosacée, de dermite atopique ou encore de dermite séborrhéique.

La Demanderesse a mis en évidence la capacité d'un extrait de bactérie filamenteuse non photosynthétique et non fructifiante cultivée sur un milieu comprenant au moins une eau minérale et/ou thermale non sulfureuse à diminuer les rougeurs cutanées.
Il a plus spécifiquement été mis en évidence que le traitement d'un explant de peau humaine en survie avec un extrait de la bactérie *Vitreoscilla filiformis* cultivée sur un milieu enrichi en eau thermale de La Roche Posay, protège le tissu d'une vasodilatation induite par la substance P.
La comparaison de cet effet avec celui d'un extrait de la même bactérie cultivée sur un milieu de culture comparable obtenu avec de l'eau distillée uniquement montre que l'extrait bactérien utilisé selon l'invention a un effet supérieur.

Ainsi, la présente invention se rapporte à l'utilisation d'au moins un extrait de bactérie filamenteuse non photosynthétique et non fructifiante cultivée sur un milieu comprenant au moins une eau minérale et/ou thermale non sulfureuse comme agent prévenant et/ou limitant et/ou corrigeant l'apparition des rougeurs cutanées.

### Extrait bactérien

L'extrait bactérien utilisé selon l'invention est préparé suivant un procédé comprenant la culture d'au moins une bactérie filamenteuse non photosynthétique et non fructifiante sur un milieu comprenant au moins une eau minérale et/ou thermale non sulfureuse.

Les bactéries utilisées sont des bactéries filamenteuses non photosynthétiques qui comprennent notamment les bactéries appartenant à l'ordre des Beggiatoales, et plus particulièrement les bactéries appartenant aux genres Beggiatoa, Vitreoscilla, Flexithrix ou Leucothrix.
Pour la mise en oeuvre de l'invention, on préfère les bactéries appartenant au genre Vitreoscilla, en particulier pour les bactéries de l'espèce *Vitreoscilla filiformis.*

Ces bactéries dont plusieurs ont déjà été décrites ont généralement un habitat aquatique, et peuvent être trouvées notamment dans des eaux marines ou dans des eaux thermales. Parmi les bactéries utilisables, on peut citer par exemple :
*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)
De préférence, la bactérie est celle correspondant à la souche déposée à l'ATCC sous le n° 15551.

Par eau thermale, on entend une eau chaude ou froide qui est utilisée pour ses vertus thérapeutiques ou pour un usage balnéaire. On peut utiliser une eau thermale ou une eau minérale. En général, une eau minérale est propre à la consommation, ce qui n'est pas toujours le cas d'une eau thermale. Chacune de ces eaux contient, entre autre, des minéraux solubilisés et des oligoéléments. Ces eaux sont connues pour être employées à des fins de traitement spécifique selon les oligo-éléments et les minéraux particuliers qu'elles contiennent.

De préférence, on utilise une eau thermale et/ou minérale qui présente une minéralisation supérieure ou égale à 400 mg/l.
On entend, dans l'invention, par "minéralisation", la somme des concentrations en anions et en cations présents dans l'eau thermale ou minérale. Dans les eaux thermales ou minérales utiles selon l'invention, la minéralisation est généralement comprise entre 400 et 900 mg/l.

L'eau thermale et/ou minérale utilisée selon l'invention peut avoir une minéralisation d'au moins 700 mg/l et, en particulier, une concentration totale en carbonates et en bicarbonates d'au moins 150 mg/l et plus préférentiellement d'au moins 360 mg/l et notamment en carbonate et bicarbonate de sodium supérieure à 2 mg/l. La concentration en oxyde de silicium dans l'eau utilisée dans la composition selon l'invention peut être de préférence d'au moins 6 mg/l et plus préférentiellement d'au moins 9 mg/l.

L'eau thermale ou l'eau minérale utilisée selon l'invention peut être choisie parmi l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizières, l'eau de Neyrac-les-bains, l'eau de Lons le Saunier, les Eaux Bonnes, l'eau de Rochefort, l'eau de Saint Christau, l'eau des Fumades et l'eau de Tercis-les-bains.
Avantageusement, elle est choisie parmi les eaux non sulfureuses telles que l'eau de Vittel, les eaux du bassin de Vichy, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizières, l'eau de Neyrac-les-bains, l'eau de Lons le Saunier, l'eau de Rochefort, l'eau de Saint Christau et l'eau de Tercis-les-bains.

Parmi ces eaux, celles qui présentent une concentration totale en carbonates ou bicarbonates supérieure à 360 mg/l sont l'eau de Vittel, l'eau de la Bourboule, l'eau des Fumades, l'eau d'Enghien-les-bains, l'eau de la Roche-Posay, l'eau du bassin de Vichy, l'eau d'Uriage.

Parmi ces eaux celles qui présentent une concentration en carbonates ou bicarbonates comprise entre 150 mg/l et 360 mg/l sont l'eau de Digne, l'eau de Maizières, l'eau de Rochefort, l'eau de Saint-Gervais-les-bains.

Parmi ces eaux, celles qui contiennent au moins 2 mg/l de carbonate ou bicarbonate de sodium sont l'eau de la Roche Posay, l'eau de Vittel, les eaux du bassin de Vichy, l'eau d'Uriage.

Les eaux contenant au moins 9 mg/l d'oxyde de silicium sont l'eau de la Roche Posay, l'eau de Vittel, les eaux du bassin de Vichy, l'eau d'Uriage.

Les eaux thermales ou minérales convenant particulièrement à la mise en oeuvre de l'invention ont une concentration en ions calcium supérieure ou égale à 100 mg/l, voire à 140 mg/l.
Selon un mode de réalisation avantageux, l'eau thermale ou minérale a une concentration en ions hydrogénocarbonates supérieure ou égale à 300 mg/l. Les hydrogénocarbonates, aussi appelés bicarbonates, sont notamment présents à une concentration supérieure ou égale à 350 mg/l.

Selon un mode de réalisation avantageux, les bactéries sont cultivées sur un milieu comprenant au moins une eau thermale. Celle-ci peut en particulier être choisie parmi l'eau de Vittel, les eaux du bassin de Vichy, l'eau d'Uriage, l'eau de la Roche-Posay, l'eau de la Bourboule, l'eau des Fumades, l'eau d'Enghien-les-bains, les Eaux Bonnes, et notamment parmi l'eau de Vittel, les eaux du bassin de Vichy, l'eau de la Roche-Posay, l'eau de la Bourboule et l'eau d'Enghien-les-bains.

Les eaux permettant d'obtenir un résultat particulièrement avantageux selon l'invention sont notamment choisies parmi l'eau de la Roche Posay et l'eau de Vittel, ou une eau de composition similaire.
L'eau de la Roche Posay est extraite de la source du même nom, il s'agit d'une eau bicarbonatée calcique, silicatée et séléniée. Elle comprend généralement environ 387 mg/l d'ions bicarbonates, environ 140 mg/l d'ions calcium et au moins 4 mg/l de sulfates.
L'eau de Vittel est riche en calcium et sels minéraux (841 mg/l) et contient notamment 202 mg/l de calcium, 402 mg/l de bicarbonates et 336 mg/l de sulfates.

On peut en particulier effectuer une culture dans le milieu suivant :

| **Composition** | **Concentration** |
|---|---|
| Extrait autolytique de levure | 0,5 à 5 g/l |
| Peptone végétale | 0,5 à 5 g/l |
| Glucose anhydre | 0,5 à 7 g/l |
| Micro-éléments de Heller | 0,5 à 5 ml/l |
| CaCl₂, 10 H₂O | 0,010 à 0,200 g/l |

On complète à 1000 ml par de l'eau minérale et/ou thermale éventuellement complétée d'eau distillée ou osmosée.

Parmi les peptones utilisables, on peut citer par exemple la peptone papaïnique de soja.
Ce milieu se distingue des milieux généralement utilisés par l'absence de catalase et de sulfure.
Les micro-éléments de Heller ont été décrits par Heller, Ann Sci. Nat. Biol. Veg. 14 : 1-223 (1953). Il s'agit de mélanges de divers éléments minéraux qui ont été recommandés par Heller, non pas pour la culture des bactéries, mais pour la nutrition des tissus végétaux cultivés *in vitro.*

La culture peut être effectuée à la température appropriée convenant pour l'espèce bactérienne cultivée. Généralement cette température est comprise entre 18 et 40°C suivant les souches. Le pH du milieu de culture est de préférence compris entre 5,5 et 8.

La composition des micro-éléments de Heller, pour 1 I d'eau, est la suivante:

| | |
|---|---|
| ZnSO₄,7 H₂O | 1 g |
| MnSO₄,H₂O | 0,076 g |
| CuSO₄,5H₂O | 0,003 g |
| Kl | 0,010 g |
| H₃BO₃ | 1 g |
| AlCl₃,6H₂O | 0,050 g |
| NiCl₂,6H₂O | 0,030 g |

Lesdites eaux thermales ou minérales peuvent représenter tout ou partie de la phase aqueuse du milieu de culture. Elles peuvent ainsi se trouver en mélange en toute proportion avec l'eau, en particulier distillée ou osmosée, présente dans le milieu de culture. Le mélange (i) d'eau thermale et (ii) d'eau osmosée ou distillée pourra être dans un ratio compris entre 0,1% et 100%, notamment de 0,1 à 50, en particulier de 0,1 à 25.
En particulier, un extrait de bactérie filamenteuse convenant à l'utilisation selon l'invention est susceptible d'être obtenu par culture de ladite bactérie sur un milieu de culture qui contient un mélange (i) d'eau osmosée ou distillée et (ii) d'eau thermale, dans un ratio (i)/(ii) compris entre 1 et 100, notamment de 1 à 50, en particulier de 1 à 25.
On utilisera en particulier l'eau thermale de La Roche Posay, telle que définie dans ce qui précède.

Après mélange de tous les éléments du milieu, on procède avantageusement à une stérilisation du milieu de culture contenant l'eau thermale et/ou minérale; cette étape est effectuée par des méthodes connues de l'homme du métier telles que la stérilisation par filtration ou par la chaleur.

Le milieu de culture est ensuite ensemencé par les bactéries.

Les milieux convenant le mieux à la culture des bactéries sont tels que l'eau thermale ou minérale représente de préférence au moins 0,1% de la quantité d'eau introduite pour la préparation du milieu, notamment de 0,1 à 99,9%. De bons résultats sont obtenus avec des concentrations d'eau thermale d'environ 1 ou 2%, notamment d'environ 1,33% par rapport à l'eau osmosée et/ou distillée, par exemple de 0,5 à 20%, voire de 0,5 à 50%, mais ces concentrations peuvent être augmentées sans inconvénient.

De façon connue, le procédé de préparation de l'extrait bactérien comprend au moins une étape dans laquelle on récupère les bactéries à la fin de la culture, en particulier en les séparant du milieu de culture.

Après culture des bactéries, on peut isoler la biomasse par diverses méthodes connues, par exemple par filtration, par coagulation avec un alcool (éthanol, isopropanol, isobutanol), par séchage sur cylindre à précouche (amidon, diatomées...) raclée, ou par lyophilisation. Une concentration préalable, par exemple à 80°C sous pression réduite, améliore cette séparation.
La biomasse peut être utilisée vivante ou bien être traitée par différents procédés. On peut procéder à une opération de rupture des enveloppes, par exemple par l'action des ultrasons. On peut en outre préparer des extraits à l'aide d'un alcool tel que l'éthanol ou le propanol.
On peut également préparer des extraits lipopolysaccharidiques selon les méthodes connues, voir par exemple Noris et Ribbons, Methods in Microbiology, Vol. 5B, Academic Press (1971). La méthode généralement utilisée est la méthode bien connue dite de Westphal (ou une méthode apparentée), qui consiste à faire l'extraction avec des mélanges phénol-eau à 65°C. On soumet ensuite l'extrait à une dialyse pour éliminer le phénol.

L'extrait bactérien utile selon l'invention peut encore résulter de la mise en oeuvre du procédé suivant : (i) on cultive au moins une bactérie appartenant à l'ordre des Beggiatoales sur un milieu comprenant un ose comme source principale de carbone et au moins une eau minérale ou thermale, puis (ii) après fermentation, on sépare les bactéries du milieu de culture, pour récupérer ladite masse des bactéries.

Les bactéries récupérées à l'issue de l'étape de fermentation peuvent notamment être soumises à un traitement de stabilisation et/ou d'extraction. C'est l'extrait de bactéries filamenteuses ainsi obtenu qui sera généralement utilisé dans ou pour la préparation de compositions cosmétiques ou dermatologiques. De manière connue en soi, l'extrait pourra ainsi être stérilisé en particulier par filtration ou par autoclavage.

Par extrait de bactéries filamenteuses non photosynthétiques, on entend aussi bien le surnageant de culture desdites bactéries, la biomasse obtenue après culture desdites bactéries ou encore les extraits de la biomasse obtenus par traitement de cette biomasse.
Pour préparer l'extrait selon l'invention, on peut cultiver lesdites bactéries selon le procédé selon l'invention, puis les séparer de la biomasse obtenue, par exemple par filtration, centrifugation, coagulation et/ou lyophilisation.

Ainsi, après culture, les bactéries sont concentrées par centrifugation. La biomasse obtenue est autoclavée. Cette biomasse peut être lyophilisée pour constituer ce que l'on appelle l'extrait lyophilisé. Toute méthode de lyophilisation connue de l'homme du métier est utilisable pour préparer cet extrait.
La fraction surnageante de cette biomasse peut également être filtrée dans un récipient stérile pour éliminer les particules en suspension. Cette fraction surnageante peut également être transvasée stérilement dans un récipient stérile. Selon un mode de réalisation particulier de l'invention, la fraction surnageante ainsi obtenue est utilisée à titre d'actif cosmétique ou dermatologique.

L'extrait bactérien utile selon l'invention peut être formulé dans un support approprié à raison d'au moins 20% en poids par rapport au poids total de la composition, en particulier à raison de 0,001 à 20% en poids par rapport au poids total de la composition et plus particulièrement à raison de 0,01 à 10% en poids par rapport au poids total de la composition.
Pour certaines applications ou formulations spécifiques, il peut être avantageux d'utiliser des concentrations pondérales élevées en extrait bactérien, par exemple, comprises entre 15 à 20%.

L'extrait bactérien selon l'invention peut-être utilisé sous une forme poudre lyophilisée, par exemple, qui peut atteindre des % de l'ordre de 0,001% à 20%, en poids d'extrait sec de bactéries filamenteuses non fructifiantes non photosythétiques par rapport au poids de la composition. Dans des formes d'application particulières de type balnéothérapie, on peut également envisager des applications en des proportions supérieures.

L'extrait bactérien cultivé sur un milieu enrichi en eau thermale peut encore être utilisé sous forme de fractions de composants cellulaires ou sous la forme de métabolites. Le(s) microorganisme(s), métabolite(s) ou fraction(s) peu(ven)t également être introduit(s) sous la forme d'une poudre lyophilisée, d'un surnageant de culture et/ou le cas échéant sous une forme concentrée.

Pour certaines applications, la biomasse vivante peut être utilisée telle qu'elle par exemple sous forme de masques ou de cataplasme pour produire un effet immédiat.

Au sens de l'invention, le terme "métabolite" désigne toute substance issue du métabolisme des microorganismes considérés selon l'invention et dotée d'une efficacité pour le traitement des rougeurs de la peau.

De manière inattendue, la Demanderesse a constaté que les extraits bactériens cultivés sur eau thermale pouvaient s'avérer efficaces pour le traitement des rougeurs de la peau.

En effet, la Demanderesse a pu mettre en évidence que l'extrait de la bactérie *Vitreoscilla filiformis* cultivée sur l'eau thermale de la Roche Posay a une efficacité de traitement des rougeurs cutanées accrue par rapport à l'extrait de la même bactérie cultivée sur milieu classique, c'est-à-dire sans eau minérale ou thermale.
La différence essentielle entre ces deux extraits réside dans les protocoles de préparation du milieu de culture où il y a substitution, au moins en partie, de l'eau osmosée par l'eau de La Roche Posay. Cela conduit notamment à une modification du métabolisme des bactéries causée par un enrichissement de du milieu de culture en éléments minéraux, particulièrement en sélénium, strontium et zinc.

Il est également intéressant de noter que l'introduction de cette biomasse dans un support formulatoire n'entraîne pas de risque de surexposition à ces éléments, Se et Zn sont des éléments essentiels à l'organisme et le Sr est largement répandu dans l'alimentation.
Le tableau ci-dessous fournit les concentrations de ces éléments chimiques dans l'extrait bactérien selon l'invention préparé selon le protocole de l'exemple 1 (extrait lyophilisé).

| | |
|---|---|
| Se (mg/kg) | 6 |
| Sr (mg/kg) | 10 |
| Zn (mg/kg) | 216 |

Ainsi, l'application de cet extrait enrichi entraîne des expositions topiques en sels minéraux par jour de l'ordre de :

| | |
|---|---|
| Se (µg/J) | 0,008 |
| Sr (µg/J) | 0,0032 |
| Zn (µg/J) | 0,094 |

Rappelons ici que l'utilisation des ions pour l'amélioration de l'état cutané est très ancienne. Ainsi, les cures thermales à visée dermatologique sur les bords de la Mer Morte -étendue d'eau la plus saline du monde- remontent à l'Antiquité (Abels DJ et coll, Clinics in Dermatol 14 : 653-658,1996). Ces bains exercent une activité anti-prurigineuse et il n'est pas rare que les personnes traitées éprouvent le sentiment d'avoir une peau plus lisse et souple (Even-Pazz Z, Isr J Med Sci 32 :11-15, 1996). A ce jour, l'intérêt de l'application topique de cations a été autant étudié dans le domaine de la sensibilité que dans celui de la sécheresse cutanée. Parmi les cations divalents, c'est l'effet apaisant du strontium qui a été le plus documenté (Hahn GS, In biochemical modulation of skin reactions. Kydonieus AF, Will JJ (eds), CRC, Boca Raton, Florida, US, 261-272, 2000).

Plus spécifiquement, la présente invention se rapporte à l'utilisation cosmétique d'au moins un extrait de bactérie non photosynthétique et non fructifiante cultivée sur une eau minérale et/ou thermale non sulfureuse comme agent pour traiter les rougeurs cutanées.

Par rougeur, on entend une coloration rosée à rouge, voire rouge foncée, de toute ou partie de la peau du corps, du cuir chevelu, des muqueuses ou semi-muqueuses. Cette manifestation, encore appelée érythème, qui peut être un signe de bonne mine (joues roses) est le plus souvent indésirable en particulier lorsqu'elle est associée à d'autres symptômes d'un désordre cutané tel que la rosacée, la dermite atopique ou encore la dermite séborrhéique.

Par traiter, sauf indication contraire, on entend toute action visant à améliorer le confort, le bien-être d'un individu, ce terme couvre donc aussi bien prévenir, atténuer, soulager que curer.

L'invention vise donc l'utilisation d'au moins un extrait de bactérie filamenteuse non photosynthétique et non fructifiante cultivée sur un milieu comprenant au moins une eau minérale et/ou thermale non sulfureuse comme agent destiné à traiter les rougeurs cutanées.

Ce traitement peut viser aussi bien les peaux saines que les peaux qui sont le siège d'une pathologie présentant un désordre du système cutané vasculaire.

Plus spécifiquement, l'invention se rapporte à l'utilisation de l'extrait bactérien comme agent destiné au traitement des rougeurs cutanées, des flushes et des érythèmes paroxystiques.

L'invention se rapporte également à l'utilisation d'au moins un extrait de bactérien filamenteuse non photosynthétique et non fructifiante cultivée sur un milieu comprenant au moins une eau minérale et/ou thermale non sulfureuse pour embellir l'aspect de la peau et/ou des muqueuses, prévenir et/ou atténuer l'intensité des rougeurs cutanées et/ou les oedèmes cutanés légers, éclaircir et/ou uniformiser le teint et/ou masquer les rougeurs de surface et/ou d'estomper les signes de la microcirculation cutanée, c'est-à-dire rendre moins visibles les microvaisseaux sanguins apparents notamment sur le visage.

L'invention se rapporte également à l'extrait de bactérie filamenteuse non photosynthétique et non fructifiante cultivée sur un milieu comprenant au moins une eau minérale et/ou thermale non sulfureuse pour son utilisation pour le traitement des rougeurs de la rosacée.
L'invention se rapporte encore à l'utilisation d'au moins un extrait de bactérie filamenteuse non photosynthétique et non fructifiante cultivée sur un milieu comprenant au moins une eau minérale et/ou thermale non sulfureuse pour la préparation d'une composition destinée au traitement des rougeurs associées à une pathologie cutanée, par exemple, aux rougeurs de la rosacée.

La rosacée est une dermatose commune chronique et progressive liée à une relaxation vasculaire. Elle affecte principalement la partie centrale du visage et se caractérise par le rougissement du visage ou les bouffées de chaleur, l'érythème facial, les papules, les pustules, et la télangiectasie. Dans les cas graves, particulièrement chez l'homme, le tissu mou du nez peut enfler et produire un gonflement bulbeux appelé rhinophyma.
La rosacée évolue généralement en 4 stades :
- stade 1 des relaxations vasculaires ayant une composante neurogène (vers 20 ans). Les patients ont des poussées soudaines de rougeur paroxystique du visage et du cou, avec sensation de chaleur, mais sans signes systémiques. Après les crises, la peau du visage redevient normale. Ces « flushes » sont déclenchés par les changements de température (entraînant parfois une thermophobie), l'absorption de boissons chaudes ou d'alcool.
- stade 2 érythémato-télangiectasique (vers 30 ans). Les zones malaires sont diffusément rouges. On y observe des capillaires dilatés constituant la classique couperose. A la différence du stade 1, la rougeur est permanente. Outre les joues, le menton et la partie médiane du front peuvent être touchés.
- stade 3 des papulo-pustules (vers 40 ans). Sur un fond d'érythème se développent des papules et des pustules de quelques millimètres de diamètre, sans comédons associés. Cette dermatose peut être très étendue, parfois à la partie glabre du cuir chevelu chez l'homme, mais respecte le pourtour de la bouche et des yeux. Les patients se plaignent d'une peau sensible, avec intolérance subjective à la plupart des topiques et des cosmétiques gras.
- stade 4 du rhinophyma (vers 50 ans ou plus tard). Cette phase tardive touche principalement les hommes, contrairement aux autres stades. Le nez est augmenté de volume, diffusément rouge et les orifices folliculaires sont dilatés. La peau s'épaissit progressivement.

L'utilisation selon l'invention est particulièrement adaptée au traitement des rougeurs cutanées du premier stade de la rosacée.

L'invention présente aussi un intérêt pour le traitement des érythèmes cutanés provoqués par des agents externes qu'il s'agisse de produits susceptibles de provoquer une irritation cutanée ou d'agents atmosphériques (exposition à la lumière, au vent au froid, à l'air sec, aux variations brutales de températures...).

L'utilisation selon l'invention se rapporte encore à la préparation de compositions destinées au traitement des érythèmes solaires, des rougeurs accompagnant une brûlure, des érythèmes associés à la dermatite atopique et des érythèmes associés à la dermite séborrhéique.
L'invention se rapporte donc aussi à l'extrait de bactérie filamenteuse non photosynthétique et non fructifiante cultivée sur un milieu comprenant au moins une eau minérale et/ou thermale non sulfureuse pour son utilisation pour le traitement des érythèmes solaires, des rougeurs accompagnant une brûlure, des érythèmes associés à des désordres cutanés, la dermatite atopique et/ des érythèmes associés à la dermite séborrhéique.

Selon un aspect préféré de l'invention, on utilise une quantité efficace d'un extrait de bactérie filamenteuse non photosynthétique et non fructifiante cultivée sur un milieu comprenant au moins une eau minérale et/ou thermale non sulfureuse pour la préparation d'une composition destinée à traiter les érythèmes associés à des désordres cutanés, tels que l'urticaire, les dermatites eczémateuses, le psoriasis, l'herpes, les photodermatoses, la dermatite de contact, le lichen, les prurigos, les maladies prurigineuses, les fibroses, les troubles de la maturation du collagène, la sclérodermie, l'eczéma.

La présente invention a également pour objet un procédé de traitement cosmétique, caractérisé en ce que l'on applique une quantité efficace d'au moins un extrait de bactérie filamenteuse non photosynthétique et non fructifiante cultivée sur un milieu comprenant au moins une eau minérale et/ou thermale non sulfureuse sur la peau et/ou le cuir chevelu et/ou sur les muqueuses et semi muqueuses.

En particulier, il pourra s'agir d'un procédé cosmétique pour prévenir et/ou estomper les rougeurs et/ou les signes de la microcirculation cutanée, caractérisé en ce que l'on applique une quantité efficace d'au moins un extrait de bactérie filamenteuse non photosynthétique et non fructifiante cultivée sur un milieu comprenant au moins une eau minérale et/ou thermale non sulfureuse sur la peau et/ou le cuir chevelu et/ou les muqueuses et semi muqueuses.

L'extrait bactérien selon l'invention est utilisé pour la préparation de compositions, de préférence topiques, destinées au traitement de désordres cutanés présentant une composante érythémateuse, notamment la rosacée, en particulier du premier stade de la rosacée.

Quelle que soit sa destination, l'extrait bactérien selon l'invention est administré selon la voie la plus appropriée à l'effet recherche, notamment par voie orale ou topique, de préférence par voie topique.
Par voie topique, on entend une administration des extraits selon l'invention ou des compositions qui le comprennent par application sur la peau.

Sauf indication contraire, dans le cadre de l'invention, par peau, on entend toute surface cutanée du corps incluant la peau et élargie au cuir chevelu et aux muqueuses et semi-muqueuses et par phanères, on entend les cils, poils, cheveux et ongles.

Les compositions topiques selon l'invention qui sont destinées au traitement de la peau, des muqueuses et semi-muqueuses et du cuir chevelu, peuvent se présenter sous forme d'onguent, de crèmes, de laits, de pommades, de poudres, de tampons imbibés, de solutions, de gels, de sprays, de lotions, ou de suspensions. Elles peuvent également se présenter sous forme de microsphères ou nanosphères ou de vésicules lipidiques ou polymériques ou de patchs polymériques et d'hydrogels permettant une libération contrôlée. Ces compositions par voie topique peuvent se présenter soit sous forme anhydre, soit sous forme aqueuse.

Les compositions sont notamment destinées à l'hygiène capillaire. Elles peuvent se présenter notamment sous forme d'une crème, d'un lait, d'une lotion, d'un gel, de microsphères ou de nanosphères ou vésicules lipidiques ou polymèriques, d'un savon ou d'un shampoing.

Dans les compositions selon l'invention, l'extrait de bactérie filamenteuse non photosynthétique et non fructifiante cultivée sur une eau minérale et/ou thermale non sulfureuse est avantageusement utilisé en association avec des rétinoïdes ou des corticostéroïdes, ou associé avec des anti-radicaux libres, avec des alpha-hydroxy ou alpha-céto acides ou leurs dérivés, ou encore des bloqueurs de canaux ioniques.

Les compositions dermocosmétiques selon l'invention peuvent, en outre, contenir des additifs inertes ou même pharmacodynamiquement ou cosmétiquement actifs ou des combinaisons des ces additifs et notamment : des agents mouillants, des agents dépigmentant tels que l'hydroquinone, l'acide azelaïque, l'acide caféïque ou l'acide kojique ; des émollients ; des agents hydratants comme le glycérol, le PEG-400, l'urée ; des agents antiséborrhéiques ou antiacnéiques, tels que la S-carboxyméthylcystéine, la S-benzyl-cystéamine, leurs sels et leurs dérivés, ou le peroxyle de benzoyle ; des antibiotiques comme l'érythromycine et ses esters, la néomycine, la clindamycine et ses esters, les tétracyclines ; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-methyl 1,2,4-benzothiadiazine 1,1-dioxyde) et le Phénytoïne (5,4-diphényl-imidazoline 2,4-dione) ; des agents anti-inflammatoires non stéroïdiens, des caroténoïdes, notamment le β-carotène ; des agents anti-psoriasiques tels que l'anthraline et ses dérivés et enfin, les eicosa-5,8,11,14-tétraénoïque et eicosa-5,8,11-trynoïque, leurs esters et les amides.

La composition selon l'invention peut également contenir des agents conservateurs, tels que des esters de l'acide parahydroxybenzoïque, les agents stabilisants, des agents régulateur d'humidité, des agents émulsionnants, des filtres UVA et UVB, des anti-oxydants, tels que l'alpha-tocophérol, le butylhydroxyanisole ou le butylhydroxytoluène.

L'objet de l'invention se rapporte également à des compositions associant au moins un extrait de bactérie filamenteuse non photosynthétique et non fructifiante cultivée sur un milieu comprenant au moins une eau minérale et/ou thermale non sulfureuse avec un ou plusieurs autres actifs, notamment choisis parmi les agents agissant sur la microcirculation.

Les actifs agissant sur la microcirculation (vasoprotecteur, vasoconstricteur) peuvent être choisis parmi les flavonoïdes, les ruscogénines, les esculosides, l'escine extraite du marron d'Inde, les nicotinates, l'héperidine méthyl chalcone, les huiles essentielles de lavande ou de romarin, les extraits de Ammi Visnaga.
La quantité de ces actifs peut varier dans une large mesure. De manière générale, ces actifs sont présents en une concentration allant de 0,01 à 15 % et de préférence de 0,05 à 10 % en poids par rapport au poids total de la composition.

La composition selon l'invention peut également constituer une composition cosmétique colorée et notamment une composition de maquillage de la peau, des fibres kératiniques (cheveux ou cils) et/ou des muqueuses, en particulier un fond de teint, un blush, un fard à joues ou à paupières, un rouge à lèvres ou un brillant à lèvres, présentant éventuellement des propriétés de soin ou de traitement. De préférence, il pourra s'agir d'une composition de maquillage colorée (beige ou verte) destinée à corriger la couleur du teint et plus particulièrement estomper ou camoufler les rougeurs.

Par composition teintée masquant les rougeurs selon l'invention, on entend préférentiellement une composition teintée de couleur verte. La composition selon l'invention comprend alors un ou plusieurs agents colorants de façon à apporter à la composition la teinte verte recherchée. Le concept de la teinte verte est choisi selon le principe d'opposition des couleurs symbolisé par le cercle chromatique. L'intensité d'une couleur peut être diminuée par l'addition de sa couleur opposée sur le cercle chromatique où sont représentées toutes les couleurs, chacune ayant son opposé. Dans tous les domaines artistiques, l'unique façon de neutraliser une tache de couleur est de la mélanger avec son opposé. Le vert étant opposé au rouge sur le cercle chromatique, c'est une teinte verte qui est choisie pour colorer la composition selon l'invention afin de neutraliser les rougeurs diffuses de la peau.

Afin d'apporter la teinte verte recherchée à la composition selon l'invention, des agents colorants sont incorporés à la composition.

Par agent colorant, on entend le terme générique employé par l'homme de l'art, définissant une substance apportant une couleur à un milieu (Article de Gisbert Otterstätter : "Coloring Cosmetics, Cosmetics&toiletries magazine, vol111, March 1996, page 25-33), telle :
- les colorants définis comme solubles dans le milieu à colorer, ils sont souvent liposolubles ou hydrosolubles,
- les pigments ou laques de couleur généralement insolubles dans le milieu à colorer, les laques étant obtenus par précipitation de colorants hydrosolubles avec un sel insoluble dans l'eau tel un hydroxyde d'aluminium,
- les pigments hydrodispersibles qui, par addition d'un solvant, conduisent à des dispersions stables dans l'eau permettant leur utilisation en tant que colorants,
pris seuls ou en mélange.

A titre d'exemples non limitatifs d'agents colorants utilisables selon l'invention, on peut nommer les pigments tels les oxydes ou hydroxydes de fer, brun, jaune ou rouge, les oxydes ou hydroxydes de chrome, les oxydes ou hydroxydes de titane, les mélanges de colorants bleus et jaunes tels les laques référencées FD&C Blue n°1 aluminium Lake (E133), FD&C Blue n°2 aluminium Lake (E132), FD&C Yellow n°5 Aluminium Lake (E102), ou FD&C Yellow n°6 Aluminium Lake (E110).

Les agents colorants selon l'invention peuvent être utilisés seuls ou en mélanges afin d'apporter la teinte verte recherchée à la composition selon l'invention.

Selon un mode préféré de l'invention, la composition comprend également des oxydes de titane aux côtés d'autres agents colorants pour leur propriété couvrante.

Les agents colorants choisis selon l'invention se devront également d'être compatibles avec une application sur la peau et les muqueuses et ce sans induire d'effet indésirable.

La composition selon l'invention contient de manière préférentielle une quantité totale d'agents colorants allant de 0.001% à 10% en poids de la composition, étant entendu que, de manière préférentielle, la quantité totale d'agents colorants ne pourra excéder 1% en poids de la composition. Chaque agent colorant est utilisé dans des proportions allant de 0.0001 à 1 % en poids de la composition.

Les mélanges d'agents colorants préférés utilisés pour la composition selon l'invention sont les suivants :
- FD&C blue n°2 aluminium lake + oxyde de fer jaune + dioxyde de titane ;
- Hydroxyde de chrome + Oxyde de fer jaune + dioxyde de titane ;
- FD&C blue n°1 aluminium lake + oxyde de fer jaune + dioxyde de titane ;
- Hydroxyde de chrome + dioxyde de titane.

Dans une autre variante de l'invention, l'extrait de bactérie filamenteuse non photosynthétique et non fructifiante cultivée sur un milieu comprenant au moins une eau minérale et/ou thermale non sulfureuse est associé à un actif visant à traiter la rosacée.

### Exemple 1 - Préparation d'un extrait bactérien selon l'invention : biomasse de Vitreoscilla filiformis cultivée sur un milieu enrichi en eau thermale de La Roche Posay

### Préparation du milieu de culture :

### Composition :

| | |
|---|---|
| * Extrait de levure | 2 à 3 g |
| * Peptone Papaïnique de soja | 2 à 3 g |
| * Glucose | 2 à 3 g |
| * Micro élément de Heller | 2 ml |
| * CaCl₂, 2H2O | 66.21 mg |
| * Eau thermale La Roche Posay | 13 -14 ml. |

Cette solution mère sera diluée par de l'eau osmosée dans un rapport de 1/75 avant stérilisation.

Le pH du milieu est ajusté à 5,00 par d'une solution molaire H₃PO₄. Le milieu est stérilisé par autoclavage à 121 °C pendant 30 minutes. Après refroidissement à température ambiante, le pH est réajusté à 7,20 par rajout d'une solution molaire de KOH.

### Culture :

Après ensemencement à 1% du milieu avec la souche *Vitreoscilla filiformis* déposée à l'ATCC sous le n°15551, la culture est mise en agitation orbitale à 100 tours / min et à 26°C. Après 48 heures de croissance, la culture est centrifugée à 8000 g pendant 15 minutes. Les culots sont récupérés puis autoclavés à 121 °C pendant 30 minutes. Cette biomasse peut être utilisée pour des tests d'évaluation.

### Exemple 2 - activité de l'extrait bactérien selon l'invention (biomasse de Vitreoscilla filiformis cultivée sur un milieu enrichi en eau thermale de La Roche Posay)

### Conditions expérimentales :

La mesure de l'activité de 4 produits : biomasse de *Vitreoscilla filiformis* cultivée sur un milieu supplémenté en Sr, Se et Zn (**D8**), biomasse de *Vitreoscilla filiformis* cultivée sur un milieu enrichi en eau thermale de La Roche Posay (**B51** préparée selon l'exemple 1), biomasse de *Vitreoscilla filiformis* cultivée sur un milieu H₂0 osmosée (**A28**) et biomasse de *Vitreoscilla filiformis* cultivée sur un milieu supplémenté en Sr (**E1**) est évaluée sur un explant de peau humaine en survie auquel de la substance P est administrée.

### Préparation des peaux

8 peaux humaines de donneurs différents ont été obtenues chez des femmes après consentement (âge entre 25 et 50 ans) après chirurgie plastique et maintenues en survie *ex-vivo*.
Les fragments de peau ont été déposés dans des inserts eux-mêmes disposés en suspension au-dessus de puits de culture. Du milieu (Milieu Essentiel Minimum de Dulbecco, D-MEM) (antibiotiques, SVF) a été ajouté dans le fond des puits, un passage s'effectuant par diffusion lente entre les deux compartiments par l'intermédiaire d'une membrane poreuse (0,45 mm). 5 heures de ré-équilibrage sont nécessaires avant de débuter le protocole.

### Application du milieu nutritif et modèle expérimental d'inflammation neurogène à l'aide de substance P :

Au terme des 5 heures de ré-équilibrage, les produits ont été ajoutés en prétraitement dans le milieu de culture D-MEM à la concentration de 30%. Les fragments de peau ont alors été maintenus en culture d'organes pendant 24 heures dans une étuve à atmosphère humide, à 37°C et en présence de 5% de CO2.
A J1, le modèle expérimental d'inflammation neurogène a été réalisé en ajoutant 10 µM de substance P (SP) dans le milieu de culture. Les milieux nutritifs ont été renouvelés et 24 heures d'incubation supplémentaires ont été effectuées pour toutes les conditions.

### Une étude comparative a ainsi été réalisée entre les 6 conditions suivantes:

### Modèle de stimulation par SP en comparaison avec la peau témoin :

- peau témoin (condition de base : peau non stimulée, non traitée) avec milieu D-MEM,
- peau stimulée par la substance P à 5 µM en milieu D-MEM,
- peau stimulée par la substance P à 5 µM en milieu D-MEM + **B51**
- peau stimulée par la substance P à 5 µM en milieu D-MEM + **E1**
- peau stimulée par la substance P à 5 µM en milieu D-MEM + **A28**
- peau stimulée par la substance P à 5 µM en milieu D-MEM + **D8**

### Analyses

Les fragments de peaux ont été fixés dans le liquide de Bouin et inclus en paraffine.

### Evaluation histologique de la dégranulation des mastocytes :

Les mastocytes présents dans le derme ont été révélés en bleu-violet par la coloration au bleu de toluidine. Histologiquement, un aspect bleu-violet plus ou moins intense et granuleux des mastocytes en rapport avec la présence plus ou moins importante dans leur cytoplasme de granulations basophiles et métachromatiques contenant notamment de l'histamine a été observé.

Les mastocytes ont ensuite été comptabilisées au microscope optique (15 champs au grossissement x 40, sur 3 plans de coupe) en les classant à l'aide des 3 scores suivants :
- *score 3 :* cellules fortement basophiles avec présence d'une coloration intense bleu-violet recouvrant tout le cytoplasme ou située de façon préférentielle à un pôle de la cellule (mastocytes renfermant un nombre important de granulations basophiles).
- *score 2 :* cellules colorées de façon modérée et homogène dans tout le cytoplasme de la cellule, ou comportant un faible halo bleu-violet situé à la périphérie de la cellule (mastocytes renfermant un nombre modéré de granulations basophiles).
- *score 1 :* mastocytes dégranulés avec disparition de la coloration bleu-violet du cytoplasme ou persistance de quelques rares grains disposés de façon éparse dans le cytoplasme (mastocytes renfermant un nombre faible de granulations basophiles).

La dégranulation obtenue après application de la substance P est responsable d'une diminution ou d'une absence de la coloration au bleu de toluidine des mastocytes. Cette diminution de la coloration est en rapport avec une diminution ou une disparition quasi complète du nombre de granulations présentes initialement dans le cytoplasme des mastocytes.
Les résultats ont été exprimés de la façon suivante : pour chaque sujet, le pourcentage de mastocytes de chaque score a été calculé par rapport au nombre total de mastocytes.

### Analyses statistiques

Une moyenne a été réalisée à partir des résultats obtenus sur les 8 peaux. L'analyse statistique est effectuée par le test de Student dit de l'écart réduit ou test des échantillons appariés, avec un risque α de 5%.

### Résultats :

### Evaluation histologique de la dégranulation des mastocytes

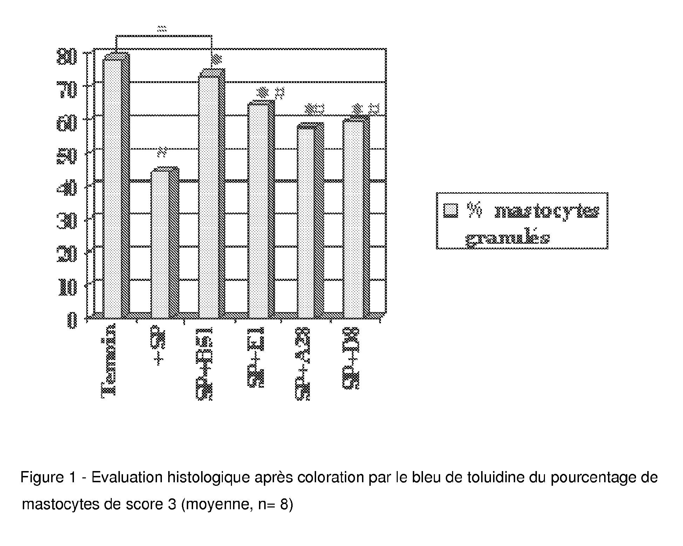

Dans le modèle de peau maintenue en survie stimulé par la SP, une diminution statistiquement significative du % de mastocytes fortement granulés (score 3) a été observés: 40% versus 80% au niveau des peaux témoins (**Figure 1** **ci-après**; p < 0,05). La substance P a donc pour effet une dégranulation des mastocytes.

Aucune différence significative entre le % de mastocytes de score 3 n'a été observée dans la condition « SP + B51 » et celui observé dans la condition « Temoin » (score de 75% versus 80%).
Cette absence de différence témoigne d'un effet protecteur de l'extrait utilisé selon l'invention (B51) vis à vis de la dégranulation induite par la substance P.
Une protection induite par les produits E1, A28, D8 est aussi observée mais elle est significativement moins importante que celle objectivé pour le produit B51.

La **figure 1** montre que les 4 préparations sont statistiquement différentes en comparaison avec l'explant de peau traitée par la Substance P. **La fraction B51 est la seule qui restaure une fonction normale.** * *p<0,05 versus peau traité* / ***dièse** p<0,05 versus peau traitée à la substance P*
# : différence statistiquement significative par rapport à la même condition sans SP (test apparié de Student, p < 0,05)
* : différence statistiquement significative par rapport au témoin (test apparié de Student, p < 0,05)

### CONCLUSION

Dans ce modèle de peau humaine maintenue en survie, un effet anti-inflammatoire de la fraction B51 utile selon l'invention a été mis en évidence.

### Exemple 3 - essais cliniques

### Données cliniques :

Une première étude clinique (étude 1), en double aveugle, a évalué en intra-individuel l'effet comparatif d'une crème contenant 5% d'extrait de *Vitreoscilla filiformis* (V. f.) cultivée de façon classique (ci-après dénommé « extrait classique » sur les rougeurs survenant chez des sujets atteints de dermatites atopiques légères à modérées (lésions symétriques contre placebo).

L'extrait dit classique de *Vitreoscilla filiformis* est préparé selon les modalités suivantes :

### - Préparation du milieu de culture :

### Composition :

| | |
|---|---|
| * Extrait de levure | 2 g |
| * Peptone Papaïnique de soja | 2 g |
| * Glucose | 2 g |
| * Micro éléments de Heller | 2 ml |
| * CaCl₂, 2H2O | 66.21 mg |
| * Eau | qsp 1I |

Le pH du milieu est alors ajusté à 5,00 par d'une solution molaire H₃PO₄. Le milieu est stérilisé par autoclavage à 121 °C pendant 30 minutes. Après refroidissement à température ambiante, le pH est réajusté à 7,20 par rajout d'une solution molaire de KOH.

### - Culture :

Au laboratoire: Après ensemencement à 1% du milieu avec la souche *Vitreoscilla filiformis,* la culture est mise en agitation orbitale à 100 tours / min et à 26°C. Après 48 heures de croissance, la culture est centrifugée à 8000 g pendant 15 minutes. Les culots sont récupérés puis autoclavés à 121 °C pendant 30 minutes. Cette biomasse peut être utilisée pour des tests d'évaluation.
En fermenteur *:* dans un fermenteur équipé préférentiellement d'un draft tube pour limiter le cisaillement, on ensemence la souche de *Vitreoscilla filiformis* à 1 % volume minimum. Le pH est maintenu stable à 7 UpH durant toute la culture, la température est régulée entre 26 et 28°C et l'oxygénation maintenue à 10% pO2 durant toute la culture par action soit sur la vitesse d'agitation soit par régulation du débit d'air. Ce type de culture peut être menée en batch, fed-batch ou en continu. Cette dernière technique est préférée car elle garantit une biomasse reproductible grâce au contrôle du taux de croissance (µ). La biomasse récoltée en continu par centrifugation à 10 000 g est congelée à -20°C. Lorsque la cuve de congélation est pleine, elle est décongelée à 4°C puis conditionnée dans des emballages manipulables par un opérateur. Ces emballages contenant la biomasse sont alors stérilisés pour être stabilisés. Chaque opération de stérilisation représente alors un lot de fabrication.

Dans le cadre de cette étude, la composition comprenant l'extrait bactérien dit « extrait classique » est appliqué 2 fois par jour ont été très bien tolérés.
L'extrait est formulé dans la composition 1A qui est une formule contenant 5% de l'extrait classique dans une émulsion huile dans eau/Arlacel/Myrj contenant 5% Parleam et 15% de silicone volatile. L'effet de cette composition 1A est comparé à celui d'un placébo : la composition 2A qui correspond à l'excipient : émulsion huile dans eau/Arlacel/Myrj contenant 5% Parleam et 15% de silicone volatile.

Cette composition 1 A contenant l'extrait « classique » de *Vitreoscilla filiformis* à 5% n'a pas d'effet significatif sur la diminution de l'érythème de la peau des sujets testés.

A contrario, dans une seconde étude (étude 2 réalisée dans les mêmes conditions que la précédente par la même équipe d'expérimentateurs), la composition contenant 5% d'extrait de *Vitreoscilla filiformis* cultivé sur un milieu enrichi en eau thermale de La Roche Posay (préparé selon l'exemple 1) à démontré également une efficacité spécifique sur l'érythème et donc sur la valence vasculaire de l'affection.
L'extrait est formulé dans la composition 1 B qui est une formule contenant 5% d'extrait bactérien selon l'invention (obtenu selon l'exemple 1) dans une émulsion huile dans eau déminéralisée Arlacel/Myrj contenant 5% Parleam, 15% de cyclopentasiloxane, 3% glycérine et 2% vaseline. L'effet de cette composition 1 B est comparé à celui d'un placébo : la composition 2B qui correspond à une émulsion huile dans eau de La Roche Posay Arlacel/Myrj contenant 5% Parleam, 15% de cyclopentasiloxane, 3% glycérine et 2% vaseline.

L'efficacité thérapeutique a été observée dans les 15 jours qui ont suivi l'application.

Ainsi, les extraits bactériens cultivés sur l'eau de la Roche Posay de la composition 1B, contrairement aux extraits bactériens connus, **ont significativement diminué l'érythème** des sujets sur les zones où ils ont été traités en comparaison à l'effet du placebo en controlatéral (**p=0, 02,** Test de Fisher).

### Exemple 4 - Exemples de Formulations

### Formule Crème

| Phases | Constituants | % (p/p) |
|---|---|---|
| A1 | Eau purifiée | QSP 100% |
| A1 | Glycérine | 4,00 |
| A1 | Sorbitol | 5,00 |
| A2 | Métronidazole | 0,75 |
| A3 | Extrait bactérien selon l'invention | 5 |
| | | |
| B1 | palmitate d'isopropyle | 2,00 |
| B1 | Cire autoémulsionnable | 12,50 |
| B2 | oxyde de fer jaune | 0,0045 |
| B2 | oxyde de chrome vert | 0,020 |
| B3 | dioxyde de Titane | 0,50 |
| | | |
| C | Alcool benzylique | 2,20 |
| | | |
| D | Acide lactique 90% | Qs pH 5 |

### PREPARATION DES PHASES - MODE OPERATOIRE

### PHASE GRASSE B

A 75°C, solubiliser sous agitation (Rayneri agitation faible) la cire autoémulsionnable dans le palmytate d'isopropyl (B1) pour conduire à l'obtention d'une phase limpide homogène.
Introduire ensuite sous agitation Rayneri le pigment jaune puis le pigment vert (B2) et le dioxide de titane (B3). Disperser pendant 30 min (Rayneri agitation modérée) pour conduire à l'obtention d'une phase verte homogène.

### PHASE AQUEUSE A

A 75°C, homogénéiser la glycérine et le Sorbitol (A1) dans l'eau purifiée sous agitation (Rayneri agitation faible).
Introduire ensuite le métronidazole (A2), vérifier sa bonne solubilisation.

Mise en émulsion à 75°C en introduisant la phase B (B1+B2) dans la phase A (Rayneri agitation modérée), maintenir cette température pendant 10 min.

Mise en refroidissement jusqu'à 45°C. Introduire l'alcool benzylique sous agitation (Rayneri agitation modérée), homogénéiser l'ensemble.

Refroidir jusqu'à 25°C sous agitation, mesurer le PH et l'ajuster si nécessaire qsp PH 5.
Ce mode opératoire conduit à l'obtention d'une crème de couleur vert clair.

### Formule Lotion

| Phases | Constituants | % (p/p) |
|---|---|---|
| A1 | Eau purifiée | QSP 100% |
| A1 | Glycérine | 7,00 |
| A2 | Carbomer 981 | 0,15 |
| A3 | PEG 400 | 2,00 |
| A4 | steareth 21 | 3,00 |
| A5 | Métronidazole | 0,75 |
| A6 | Extrait bactérien selon l'invention | 5% |
| | | |
| B1 | Oxyde de fer jaune | 0,0045 |
| B1 | Oxyde de chrome vert | 0,02 |
| B1 | dioxyde de Titane | 0,50 |
| B1 | Huile minerale | 6,00 |
| B2 | glyceryl & PEG 100 stearate | 3,00 |
| B2 | alcool stearylique | 2,00 |
| | | |
| C | Alcool benzylique | 1,30 |
| | | |
| D | Eau purifiée | 2,00 |
| D | Sorbate de potassium | 0,20 |
| | | |
| E | cyclomethicone 5 | 4,00 |
| | | |
| F | solution d'hydroxyde de Sodium à 10% | Qs pH 5 |
| F | solution à 1% d'acide lactique (90%) | Qs pH5 |

### PREPARATION DES PHASES - MODE OPERATOIRE

### PHASE GRASSE B

A 80°C, disperser sous agitation pendant 15 min. les pigments jaune et vert dans l'huile minérale (Rayneri agitation modérée). Introduire ensuite le dioxyde de titane, homogénéiser pendant 15 min. Maintenir la température à 80°C, puis introduire le stéarate de glycéryle et PEG 100, et l'alcool stéarylique (B2), homogénéiser jusqu'à solubilisation complète des cires pour obtenir une phase homogène verte.

### PHASE D

Solubiliser le sorbate de potassium dans l'eau purifiée.

### PHASE AQUEUSE A

A 80°C, disperser le Carbomer 981 (Rayneri vitesse modérée) dans l'eau purifiée et la glycérine.
Introduire le PEG 400 (A3) et le steareth 21 (A4), homogénéiser pendant 5 min.
Introduire ensuite le métronidazole (A5), vérifier sa bonne solubilisation.

Mise en émulsion à 80°C en introduisant la phase grasse B dans la phase aqueuse A sous agitation (Rayneri agitation modérée). Homogénéiser pendant 10 min.

Mise en refroidissement.
A 40°C, introduire les phases C, D, et E.

Refroidir sous agitation jusqu'à 25°C, mesurer le PH et l'ajuster si nécessaire qsp PH 5 avec la solution de soude à 10% ou l'acide lactique à 1 %.
Le mode opératoire conduit à l'obtention d'un fluide gélifié de couleur vert clair.

### Formule Crème

| Phases | Constituants | % (p/p) |
|---|---|---|
| A1 | Eau purifiée | QSP 100% |
| A1 | Glycérine | 4,00 |
| A1 | Sorbitol | 5,00 |
| A2 | Métronidazole | 0,75 |
| A3 | Extrait bactérien selon l'invention | 2 |
| | | |
| B1 | palmitate d'isopropyle | 2,00 |
| B1 | Cire autoémulsionnable | 12,50 |
| B2 | oxyde de fer jaune | 0,0095 |
| B2 | FD&C Blue Aluminium Lake | 0,0068 |
| B3 | dioxyde de Titane | 0,50 |
| | | |
| C | Alcool benzylique | 2,20 |
| | | |
| D | Acide lactique 90% QSP PH 5 | Qs pH 5 |

### Phase aqueuse

Dans un bécher, peser la glycerine, le sorbitol, l'alcool benzylique et l'eau purifiée. Introduire ensuite le métronidazole.
Faire chauffer la phase à 75°C puis solubiliser l'actif sous agitation modérée pendant 20 minutes.

### Phase grasse

Dans un bécher peser la cire autoémulsionnable et l'isopropyl palmitate. Faire chauffer la phase à 75°C, homogeneiser la phase sous agitation ultra-turrax. Introduire ensuite les pigments pesés ensemble et le dioxyde de titane sous agitation à l'ultra-turrax à 8000 tr/min pendant au moins 20 minutes.

### Emulsification et refroidissement

Mise en émulsion à 75°C pendant 10 minutes sous agitation Rayneri modérée en introduisant la phase grasse dans la phase aqueuse.
Mise en refroidissement sous agitation faible.

Obtention d'une crème épaisse verte homogène.

### Formule crème

| Phases | Constituants | % (p/p) |
|---|---|---|
| A1 | Eau purifiée | QSP 100% |
| A1 | Glycérine | 4,00 |
| A1 | Sorbitol | 5,00 |
| A3 | Extrait bactérien selon l'invention | 5 |
| | | |
| B1 | palmitate d'isopropyle | 2,00 |
| B1 | Cire autoémulsionnable | 12,50 |
| B2 | oxyde de fer jaune | 0,0045 |
| B2 | hydroxyde de chrome vert | 0,020 |
| B3 | dioxyde de Titane | 0,50 |
| | | |
| C | Alcool benzylique | 2,20 |
| | | |
| D | Acide lactique 90% QSP PH 5 | Qs pH 5 |

Le mode opératoire est le même que celui de la formulation précédente.

### Formule crème

| Phases | Constituants | % (p/p) |
|---|---|---|
| A1 | Eau purifiée | QSP 100% |
| A1 | Glycérine | 4,00 |
| A1 | Sorbitol | 5,00 |
| A3 | Extrait bactérien selon l'invention | 5 |
| | | |
| B1 | palmitate d'isopropyle | 2,00 |
| B1 | Cire autoémulsionnable | 12,50 |
| B2 | oxyde de fer jaune | 0,019 |
| B2 | FD&C Blue Aluminium Lake | 0,0136 |
| B3 | dioxyde de Titane | 1,00 |
| | | |
| C | Alcool benzylique | 2,20 |
| | | |
| D | Acide lactique 90% QSP PH 5 | Qs pH 5 |

Le mode opératoire est le même que celui de la formulation précédente.

## Revendications

1. Utilisation cosmétique d'au moins un extrait de bactérie filamenteuse non photosynthétique et non fructifiante cultivée sur un milieu comprenant au moins une eau minérale et/ou thermale non sulfureuse comme agent destiné à traiter les rougeurs cutanées.

2. Utilisation selon la revendication 1, **caractérisée en ce que** ladite bactérie est *Vitreoscilla filiformis* (souche ATCC 15551).

3. Utilisation selon la revendication 1 ou 2, **caractérisée en ce que** ladite eau est l'eau de La Roche Posay.

4. Utilisation selon l'une quelconque des revendications précédentes, **caractérisée en ce que** l'extrait est présent à raison de 0,001 à 20% en poids par rapport au poids total de la composition.

5. Utilisation selon l'une quelconque des revendications précédentes, comme agent destiné au traitement des rougeurs cutanées, des flushes et des érythèmes paroxystiques.

6. Utilisation selon l'une quelconque des revendications 1 à 4, pour masquer les rougeurs de surface et/ou d'estomper les signes de la microcirculation cutanée.

7. Utilisation selon l'une quelconque des revendications 1 à 4, comme agent destiné au traitement des érythèmes cutanés provoqués par des agents externes et/ou des agents atmosphériques.

8. Utilisation d'au moins un extrait de bactérie filamenteuse non photosynthétique et non fructifiante cultivée sur un milieu comprenant au moins une eau minérale et/ou thermale non sulfureuse pour la préparation d'une composition destinée au traitement des rougeurs cutanées.

9. Utilisation selon la revendication 8, **caractérisée en ce que** la composition est destinée au traitement des érythèmes solaires, des rougeurs liées à un désordre cutané, des rougeurs accompagnant une brûlure, des érythèmes associés à la dermatite atopique, la rosacée et/ou des érythèmes associés à la dermite séborrhéique.

10. Utilisation selon la revendication 9, **caractérisée en ce que** la composition est destinée au traitement des rougeurs cutanées du premier stade de la rosacée.

11. Utilisation selon l'une quelconque des revendications précédentes, **caractérisée en ce que** ledit extrait est associé à au moins un actif choisi parmi les rétinoïdes ; les corticostéroïdes, les anti-radicaux libres ; les alpha-hydroxy ou alpha-céto acides ou leurs dérivé ; les bloqueurs de canaux ioniques ; les agents mouillants ; les agents dépigmentant ; les émollients ; les agents hydratants ; les agents antiséborrhéiques ou antiacnéiques ; les antibiotiques ; les agents antifongiques ; les agents favorisant la repousse des cheveux ; les agents anti-inflammatoires non stéroïdiens ; les caroténoïdes ; des agents anti-psoriasiques ; les agents conservateurs ; les agents stabilisants ; les agents régulateur d'humidité ; les agents émulsionnants ; les filtres UVA et UVB ; les anti-oxydants.

12. Composition comprenant d'au moins un extrait de bactérie filamenteuse non photosynthétique et non fructifiante cultivée sur un milieu comprenant au moins une eau minérale et/ou thermale avec un agent actif choisi parmi les agents agissant sur la microcirculation vasoprotecteur ou vasoconstricteur ; une substance apportant une couleur à la peau et/ou un actif visant à traiter la rosacée.

13. Composition de maquillage de couleur verte comprenant d'au moins un extrait de bactérie filamenteuse non photosynthétique et non fructifiante cultivée sur un milieu comprenant au moins une eau minérale et/ou thermale et une substance apportant une couleur verte.

14. Composition selon la revendication 12 ou 13, **caractérisée en ce que** ladite bactérie est *Vitreoscilla filiformis* (souche ATCC 15551).

15. Composition selon la revendication 12 à 14, **caractérisée en ce que** ladite eau est l'eau de La Roche Posay.

16. Composition selon l'une quelconque des revendications 12 à 15, **caractérisée en ce que** l'extrait est présent à raison de 0,001 à 20% en poids par rapport au poids total de la composition.

17. Procédé cosmétique pour prévenir et/ou estomper les rougeurs et/ou les signes de la microcirculation cutanée, **caractérisé en ce que** l'on applique une quantité efficace d'au moins un extrait de bactérie filamenteuse non photosynthétique et non fructifiante cultivée sur un milieu comprenant au moins une eau minérale et/ou thermale non sulfureuse sur la peau et/ou le cuir chevelu et/ou les muqueuses et semi muqueuses.

18. Procédé selon la revendication 17, **caractérisé en ce que** ladite bactérie est *Vitreoscilla filiformis* (souche ATCC 15551).

19. Procédé selon la revendication 17 ou 18, **caractérisé en ce que** ladite eau est l'eau de La Roche Posay.

20. Procédé selon l'une quelconque des revendications 17 à 19, **caractérisé en ce que** l'extrait est présent à raison de 0,001 à 20% en poids par rapport au poids total de la composition.
